(19) Europäisches Patentamt
European Patent Office
Office européen des brevets

(11) Publication number: 0 461 043 A2

# (12) EUROPEAN PATENT APPLICATION

(21) Application number: 91401498.0

(22) Date of filing: 07.06.91

(51) Int. Cl.$^5$: C12P 41/00, C07C 67/02, C12N 9/62

(30) Priority: 08.06.90 US 535363

(43) Date of publication of application: 11.12.91 Bulletin 91/50

(84) Designated Contracting States: AT BE CH DE DK ES FR GB GR IT LI LU NL SE

(71) Applicant: SEPRACOR, INC.
33 Locke Drive
Marlborough, MA 01752 (US)

(72) Inventor: Dodds, David R.
25 Tichonderoga Lane
Millis, MA 02054 (US)
Inventor: Zepp, Charles M.
19 Highland Street
Berlin, MA 01503 (US)
Inventor: Rossi, Richard F.
272 Reservoir Street
Norton, MA 02766 (US)

(74) Representative: Warcoin, Jacques et al
Cabinet Régimbeau 26, avenue Kléber
F-75116 Paris (FR)

## (54) Enantiomer separation by transesterification.

(57) Disclosed is a process for resolving enantiomeric mixtures of 2-substituted propanoic acids and related compounds by first derivatizing the compounds to produce water soluble ester forms of the acids, and using the ester forms in an enantiomerically selective enzyme catalyzed reaction. The water soluble substrates are disposed in an aqueous solution of a water soluble donor alcohol in the presence of a hydrolase enzyme and produce by transesterification a product ester rich in one of the two enantiomers having reduced water solubility. Separation of the enantiomers thereafter may be conducted using various conventional means. The process is versatile and efficient because it can be conducted in aqueous solution to produce an easily separated product which inherently is a relatively poor substrate for hydrolase enzyme catalyzed hydrolysis.

EP 0 461 043 A2

This invention relates to the separation of enantiomers by preferential transesterification of one enantiomer in the presence of a donor alcohol and a hydrolase enzyme.

Separations of enantiomeric mixtures can be important as small stereochemical differences about the chiral center of such compounds can lead to profoundly different properties in biological systems. Beta blockers, pheromones, prostaglandins, steroids, flavorings, fragrances, pharmaceuticals, pesticides, herbicides, and many other compounds exhibit such critical stereospecificity. Twelve of the 20 most prescribed drugs exhibit chirality. For example, Naproxen, or (S)-2-(6-methoxy-2-napthyl) propanoic acid, is one of the two most important members of the class of 2-aryl propanoic acids with non-steroidal anti-inflammatory activity. The (S) enantiomer of Naproxen is known to be 28 times more therapeutically potent than its (R) counterpart. The L form of the beta blocker propranolol is known to be 100 times more potent than the D form.

The art has developed enzymatic resolution techniques for separating enantiomers on a preparative scale. Such techniques exploit the natural stereoselective properties of enzymes, including lyases, oxidoreductases, and hydrolases such as lipases, proteases, and esterases such as chymotrypsin. Generally, naturally occurring enzymes can effect chemical change on a spectrum of substrates having stereochemical features approximating the enzymes' "natural" substrates. Enzymes typically exhibit at least some degree of chiral selectivity in that reaction on a pair of enantiomers results in a product which is enriched in one enantiomer and depleted in the other. Stated differently, the ratio of the reaction rate of the respective stereoisomers, $(V_RK_{MR})/(V_SK_{MS})$, or E value, typically is unequal to 1. Hydrolases such as lipases, proteases, and esterases are among the more attractive enzymes for use in resolutions as they are commercially available at reasonable costs, do not require expensive cofactors, and chemically change various classes of chiral compounds including alcohols, carboxylic acids, esters, amides, and amines.

Many of the more valuable stereospecific compounds exhibit low water-solubility, i.e., a solubility less than about 0.1M. For this reason, enzyme-mediated optical resolutions of such compounds previously were conducted under multiphasic reaction conditions. For example, Cambou and Kilbanov (Biotechnol. Bioeng., 26:1449, 1984) disclose an investigation of lipase mediated resolution of 2-(p-chlorophenoxy) propanoic acid in a two-phase system using a yeast lipase. The multiphase reaction system comprised an organic phase containing the racemic reactant ester, a separate phase of aqueous buffer, and enzyme immobilized on high surface area particulate material. The authors were able to produce (R)-acid in hundred gram quantities and 85% yields and at 97% chemical purity; the enantiomeric excess of (R) over (S) was found to be 96%. Three alternative approaches to resolution were compared. Enzymatic hydrolysis was judged to be preferred for acids while transesterification was the method of choice for the resolution of racemic alcohols.

Flaschel and Renken, in a report entitled "Optical Resolution of Phenylalanine By Enzymic Transesterification", (Biocatalysis In organic Media, Proceedings of an International Symposium, held at Wageningen, The Netherlands, 7-10 Dec. 1986, Elsevier Science Publishers, 1987) report the preparation of methyl ester of L-phenylalanine from racemic phenylalanine propyl ester by transesterification using chymotrypsin. The hydrogen propyl sulfate salt of phenylalanine propyl ester was treated at various pHs, temperatures, initial substrate concentrations, and volumetric proportions of methanol ranging from 7% to 25%. Chymotrypsin was shown to transesterify preferentially the L-form when the substrate concentration was maintained at low levels.

In copending U.S. application Serial No. 178,735 filed April 6, 1988 by Zepp et al., a methodology is disclosed to overcome many of the problems associated with enzymatic resolution of water insoluble substrates. In the method, highly water soluble ester forms of otherwise water insoluble racemic mixtures are used as substrates for hydrolase enzymes, in solution or immobilized on membranes, to produce by enzymatic hydrolysis enantiomerically enriched carboxylic acid forms of the esters. While this approach has significant utility, the pH of the aqueous reaction mixture can be such that appreciable amounts of the product carboxylic acid remain in the aqueous layer as carboxylate anion and are not extracted into the organic phase. Saturation concentrations of acid in the aqueous phase can inhibit enzymatic reaction thereby contributing to inefficiency in the procedure as the product can itself act as an inhibitor for the enzyme.

It is an object of this invention to provide novel methods for separating enantiomers using hydrolase enzymes to transesterify preferentially one of a pair of enantiomeric, water-soluble substrates in aqueous solution so as to produce a less soluble or water insoluble enantiomerically enriched product. Another object is to provide efficient methods of resolving enantiomers of 2-substituted propanoic acids and their derivatives. These and other objects and features of the invention will be apparent from the following description and claims.

## Summary of the Invention

As noted above, it is known that mixtures of water soluble enantiomers of certain classes of esters can be used as a substrate for enantiomerically selective hydrolase enzymes. It has now been discovered that such enzymes may be used in association with an aqueous solution of reactant esters containing a donor alcohol

to produce by transesterification a product ester of reduced water solubility relative to the substrate. These product esters can be separated readily from the water-soluble substrate. Furthermore, racemic mixtures of carboxylic acids can be derivatized chemically with hydrophilic ester groups, typically comprising a cationic or anionic moiety, to increase greatly the water solubility of an otherwise poorly water-soluble racemic or other mixture of enantiomers, thus expanding significantly the scope of use of the process. Use of the method permits one to optimize pH in an aqueous solution thereby to promote high enzymatic activity. The substantially water insoluble product ester typically is substantially ineffective as an enzyme substrate. Thus, it is not significantly enzymatically hydrolyzed, nor does it inhibit net catalytic efficiency by acting as an inhibitor of the enzyme.

Accordingly, the invention provides a method of separating enantiomers comprising the steps of providing a mixture of (R) and (S) enantiomers of a water soluble reactant ester of the formula:

```
        R2
        |
    R3--C--R1
        |
        Es
```

wherein Es is an ester group and $R_1$, $R_2$, and $R_3$ are different moieties which together with the carbon atom define a chiral center. This reactant mixture is disposed in association with a hydrolase enzyme in an aqueous reaction solution comprising a donor alcohol at a concentration sufficient and for a time sufficient to promote transesterification preferentially of at least a portion of one of the (R) and (S) enantiomers. The transesterification results in a product ester of one of the enantiomers which has reduced water solubility relative to the reactant-ester. The product ester then may be separated by known methods from the reactant ester to produce an enantiomerically enriched fraction.

In preferred embodiments, $R_1$, $R_2$, and $R_3$ in the formula set forth above are each independently hydrogen, halogen, lower alkyl, lower alkoxy, substituted lower alkyl, substituted lower alkoxy, hydroxy, thiol, nitrile, aromatic ring structures, substituted aromatic ring structures, heterocyclic ring structures, aryloxy ring structures, or substituted aryloxy ring structures. Es has the formula $-COOR_5X$ wherein $R_5$ is a hydrocarbon having 1 to 8 carbon atoms and X is a hydrophilic moiety which imparts water solubility to the ester. In one preferred subgenus of the invention, the reactant estets comprise a moiety X which is responsible for imparting water solubility to the reactant, and the protonated carboxylic acid analog of the ester is essentially water insoluble, i.e., the compound where $R_5X$ is a hydrogen atom has very low solubility. In this subgenus of compounds useful in the process of the invention, the transesterified product has quite low solubility in water, and any hydrolysis product also has low solubility. The preferred substrates are water soluble reactant esters having the formula:

```
         CH3
         |
    R3---CH
         |
         C = O
         |
         O - R5X
```

that is, a 2-substituted propanoic acid ester, wherein $R_5$ and X are the same as set forth above, and $R_3$ is halogen, an aromatic ring structure, a substitued aromatic ring structure, a heterocyclic aromatic ring structure, a substituted heterocyclic aromatic ring structure, an aryloxy ring structure, or a substituted aryloxy ring structure. Specific $R_3$ structures of useful reactant substrates include:

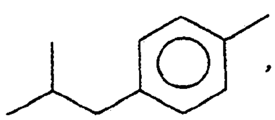 , 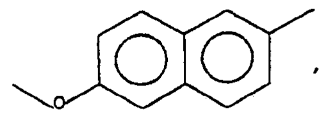 , 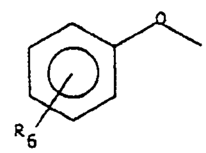

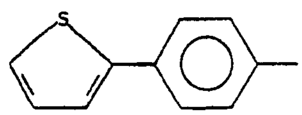 , 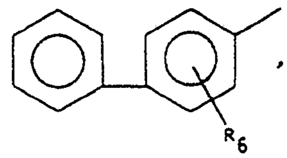 , and 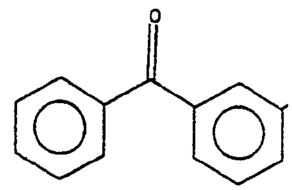

where $R_6$ is hydrogen or a halogen, e.g, fluorine or chlorine.

The symbol X represents, for example, any of the moieties disclosed in copending U.S. Patent Application Serial No. 178,735 filed April 6, 1988 (PCT 89/09765 published Oct. 19, 1989, entitled Enzymatic Resolution Systems, Compounds Useful in the Systems, and Their Preparation) and may include quaternary amines, inorganic acids, or salts thereof. Where X is a quaternary amine, it may have the formula $NZ_3$ wherein Z is hydrogen, alkyl, or aryl, and is charge balanced by a counterion, preferably a halide, carboxylate, inorganic polyatomic anion, methyl sulfate, tosylate, mesylate, or trifluoromethyl sulfonate. Preferred inorganic acids for X are $-HSO_3$, $-HSO_4$, and $-H_2PO_4$, or salts of such acids.

Specific preferred reactant substrates include water-soluble esters of 2-(p-isobutylphenyl) propanoic acid, 2 (6-methoxy-2-naphthyl) propanoic acid, 2-(aryloxy) propanoic acid, or substituted 2-(aryloxy) propanoic acids.

In preferred aspects of the invention, the donor alcohol is a water-soluble, preferably completely water miscible alcohol, and most preferably has the formula $R_4OH$ wherein $R_4$ is a hydrocarbon having 1 to 5 carbon atoms. In this case the product ester is a lower alkyl ester. The system preferably is designed such that the product ester is substantially unreactive with respect to further hydrolysis to its acid form in the presence of the hydrolase enzyme. Best results are achieved when the difference in water solubility between the reactant ester and the product ester is at least a factor of four, i.e., the ratio of water solubility between the two is greater than about 4, preferably about 10 and most preferably 100 or more. Frequently, the difference in water solubilities between the reactant and product esters can be about two orders of magnitude, e.g., the product esters have water solubilities no greater than about 1.0mM whereas the reactant esters have water solubility at least about 0.1M (100mM).

The preferred type of enzyme for use in the separation process are hydrolases in class 3.4.n.n of The Standard Rules for Classification and Nomenclature of Enzymes, including those which act on peptide bonds (peptide hydrolases). Most preferred of these is serine proteases. The currently most preferred specific enzyme is a serine protease derived from Aspergillus oryzae.

Separation of the enantiomerically enriched fractions produced by the method of the invention can be conducted by extraction in hydrophobic media, by precipitating one of the reactant or product esters, by ultrafiltration, or by other conventional means. In many instances of the process of the invention, enantiomerically selective enzymatic hydrolysis takes place to some degree simultaneously with transesterification. Typically, the product acids have relatively low water solubility (relative to the reactant esters) and can be separated together with the product ester. However, the composition of the reaction solution, e.g., the ratio of donor alcohol to water, and the concentration of reactant substrate, preferably is set as disclosed herein to promote transesterification and to minimize hydrolysis.

## Brief Description of the Drawing

The sole figure of the drawing schematically illustrates apparatus for the practice of the invention.

## Description

The invention exploits the natural stereoselectivity of enzymes to separate enantiomers in aqueous solution. More particularly, the process of the invention involves the preferential enzymatic conversion in aqueous solution of one of a pair of enantiomers in the form of a water-soluble reactant ester to the form of a water-insoluble product ester, thereby permitting easy separation by differential solubilization or other means of a fraction rich in one of the enantiomers. The reaction is conducted in the presence of a donor alcohol which is at least partially water-soluble and preferably miscible with water in all proportions. The substitution during reaction of the alcohol moiety for the ester moiety serves to decrease the water-solubility of the reactant substrate. Because the substitution reaction, called transesterification, is conducted enzymatically, and because the characteristic reaction rate of an enzymatic change differs between a given pair of enantiomers, the reaction produces fractions rich in one enantiomer having solubilities in various solvents which differ from the parent compound.

The process is efficient because it can be conducted in an aqueous solution having a pH, salt concentration, etc. designed to optimize enzymatic activity. Furthermore, the product ester, being of reduced water solubility, serves readily neither as a substrate for the enzyme, nor as an inhibitor. Furthermore, the product ester may physically separate from the aqueous reaction mixture as an insoluble phase, e.g., an oil or solid material. Thus, product is continually "removed" from the reaction solution, forcing reaction equilibrium to favor transesterification. This aspect of the invention can be exploited further by use of reactor system designs which continuously or periodically extract products of low water solubility from the reaction solution.

The process is versatile because there are a large number of acidic organic compounds of low water solubility having a chiral center alpha to the carboxylic acid group which can be esterified with a charged alcohol moiety that enhances the water solubility of the resulting substrate ester. There also are a large number of commercially available hydrolase enzymes, each of which can hydrolyze or transesterify an ester bond, depending on reaction conditions and the nature of the substrate, and which display at least some stereoselectivity. There also are a number of system configurations useful with the process of the invention. Thus, the enzyme may be free in solution, disposed at an interface between two phases, immobilized on an insoluble support structure, or contained within the pores of a membrane. The reaction may be conducted in the presence of a hydrophobic solvent as a separate phase which can extract product ester as it is produced, and this separate phase may be in direct contact with the reaction solution in the form of a water-in-oil or oil-in-water emulsion, or it may be separated from the aqueous phase by a membrane.

Broadly, the invention is practiced by reacting a mixture of (R) and (S) enantiomers of a water soluble ester having a chiral center with a donor alcohol in water in the presence of a hydrolase enzyme to effect an asymmetric transesterification that may be symbolized as follows:

$$R'COOR'' + R'''OH \xrightarrow[H_2O]{} R'COOR''' + R''OH$$

[reactant] [donor alcohol] [product ester]

where R′ contains the chiral center. The reactant is water soluble, typically but not necessarily because of the influence of the R″ ester group. The donor alcohol is also water soluble, and preferably is methanol, ethanol, n-propanol, isopropanol, butanol, ethylene glycol, methoxy ethanol, or pentanol, most preferably methanol or ethanol. Other water soluble alcohols may be used. The donor alcohol is transferred enzymatically to the reactant substrate to produce the product ester, which, relative to the reactant, is of reduced water solubility, and preferably having a water solubility low enough such that it can be considered water insoluble (i.e., a water solubility of 0.1M or less).

While the reactant substrate may be characterized herein as "water soluble" and the product ester as "water insoluble", it should be appreciated that these are relative terms. The important aspects are that the product ester be less hydrophilic than the reactant substrate, that the resulting difference in water solubility be exploitable to separate product ester from reactant ester as enantiomerically enriched species, and that the less water soluble product ester be less prone to further enzymatic reaction, i.e., hydrolysis, than the reactant substrate.

Various specific aspects relevant to the scope of the invention and how to make and use it are disclosed below.

## Reactant Esters

Broadly, the process of the invention may be practiced using water soluble forms of esters having the formula:

```
     R2
     |
R3--C--R1
     |
     Es
```

wherein Es is an ester group and $R_1$, $R_2$, and $R_3$ are different moities which together with the carbon atom define a chiral center. $R_1$, $R_2$, and $R_3$ may be, for example, hydrogen, halogen such as chlorine or fluorine, lower alkyl, e.g., a $C_1$ to $C_{10}$ linear hydrocarbon, lower alkoxy, substituted lower alkyl, substituted lower alkoxy, hydroxy, thiol, nitrile, an aromatic ring structure, a substituted aromatic ring structure, a heterocyclic ring structure, an aryloxy ring structure, or a substituted aryloxy ring structure. As can be appreciated from a review of the foregoing list, the majority of these radicals have significant hydrophobic character. Thus, the acid form of reactant substrates useful in the process of the invention (i.e., the structure wherein $E_S$ is COOH) typically has low water solubility, e.g., less than about 1 mM to 10 mM. The ester group, Es, typically will have the formula: $COOR_5X$ wherein $R_5$ is a hydrocarbon having about 1 to 8 carbon atoms, such as a linear or aromatic moiety, and X is a hydrophilic moiety which imparts water solubility to the ester. To maximize water solubility, it is preferred that $R_5$ be a methyl or ethyl group. X typically is a charged moiety, either anionic or cationic, charge balanced by a water soluble counterion. X may be, for example, a quaternary amine, inorganic acid, or a salt thereof. Bonded to the quaternary nitrogen atom of the quaternary amine may be hydrogen, an alkyl group; or an aryl group. Potentially useful counterions neutralizing the quaternary ammonium nucleus include halide, carboxylate, and inorganic polyatomic anions such as sulfate, phosphate, nitrate, and the like, methyl sulfate, tosylate, mesylate, and trifluoromethyl sulfonate. Where X is an inorganic acid group, it preferably comprises a sulfonate group, a sulfate group, a phosphate group, or a phosphonate group. Alkali metal or other salts of such acid groups also may be used.

The currently most preferred genus of compounds useful in the practice of the invention have the formula:

```
       CH3
       |
R3---CH
       |
       C = O
       |
       O - R5 -X
```

wherein $R_3$ is a halogen, an aromatic ring structure, a substituted aromatic ring structure, a heterocyclic aromatic ring structure, a substituted heterocyclic aromatic ring structure, an aryloxy ring structure, or a substituted aryloxy ring structure, and $R_5$ and X have the same meaning noted above. This class of 2-substituted water soluble propanoic acid esters if preferred because it includes a number of known valuable stereospecific compounds including the subclass of non-steroidal anti-inflammatory drugs (NSAIDs) including the (S) enantiomer of 2-(6-methoxy-2-naphthyl) propanoic acid known as Naproxen, 2-(p-isobutylphenyl) propanoic acid known as Ibuprofen, 2-(3-benzoylphenyl) propanoic acid known as Ketoprofen; and 2-(2-fluoro-4-biphenylyl) propanoic acid known as Flurbiprofen. Practice of the invention can be used to resolve 2-halosubstituted propanoic acids such as 2-chloropropanoic acid and 2-bromo-propanoic acid. It may also be used to resolve aryloxy propanoic acids such as 2-(4-chlorophenoxy) propanoic acid, 2-(2-methyl-4-chlorophenoxy) propanoic acid, and 2-(4-hydroxyphenoxy) propanoic acid. In addition, the process can be used to resolve 2-methyl-3-thio propanoic acids, 2-hydroxy-4-phenyl butanoic acid, and 2-halo-4-phenyl butanoic acids such as 2-bromo 4-phenyl butanoic acid. The structural formulae of the acid form of the currently preferred reactant esters is set forth below:

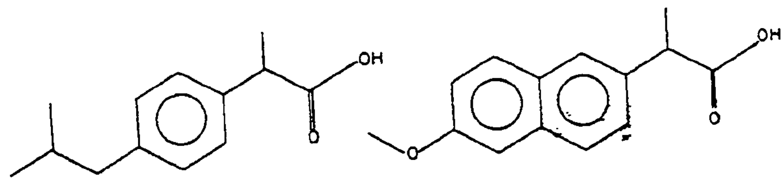

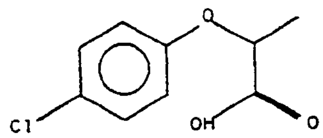

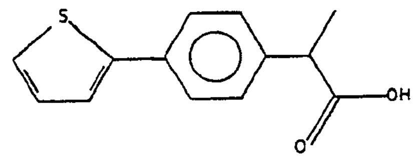

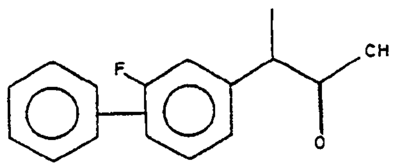

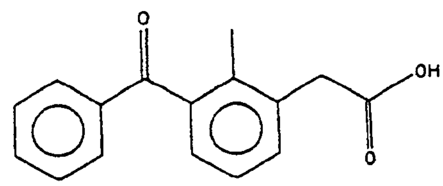

Water soluble esters of compounds of the type set forth above may be synthesized in accordance with the procedure disclosed in copending U.S. Application Serial No. 178,735 filed April 6, 1988 (corresponding PCT US/89/09765 published Oct. 19, 1989) entitled Compounds Useful in Enzymatic Resolution Systems and Their Preparation, the disclosure of which is incorporated herein by reference. Generally, the techniques employed for synthesis of such esters are well known to the organic chemist. They involve reacting the carboxylate ion of the compound with, for example, a hydroxy alkyl sulfonic acid sultone, hydroxy alkyl or hydroxy aryl sulfonic acid salt, or hydroxy terminated ester groups followed by sulfonating agents or phosphorylating agents or derivatives. Alternatively, the carboxylate ion may be reacted with an acid chloride of an inorganic acid to form the acid chloride form of the compound and thereafter with a tertiary amine alkanol followed by an alkylating agent. Other methods will be apparent to those skilled in the art.

The effect and purpose of derivatization of the desired compounds is to produce water soluble ester forms useful as reactant substrates in the process of the invention. During the process of transesterification, the alcohol moiety ether linked to the carbonyl group of the ester is replaced by the donor alcohol, for example, a methanol or ethanol group. While, as disclosed above, in the preferred practice of the invention the donor alcohol is water soluble, the effect of the transesterification is to produce a product ester of very significantly lower water solubility. For example, the methyl ester of Naproxen which is produced when methanol is the donor alcohol has a solubility in water on the order of 0.1 to 0.2 mM, the octyl ester of 2-chloropropanoic acid has a water solubility of about 1.2 mM, and the methoxy-ethyl ester of Ibuprofen has a water solubility of approximately 0.18 mM. In contrast, the methyl sulfate salt of N,N,N-trimethylammonium ethyl ester of Ibuprofen has a water solubility of 2.18 M, the sodium salt of the sulfomethyl ester of Ibuprofen has a water solubility of 1.66 M, the potassium salt of sulfoethyl ester of Naproxen has a water solubility of 12.1 mM, and the methyl sulfate salt of N,N,N-trimethylammonium) ethyl ester of Naproxen has a water solubility of 1.61 M. The acid forms of Naproxen and Ibuprofen are characterized by a water solubility at room temperature generally in the single digit mM range which varies with pH. It is an important feature of the invention that the transesterification product has not only a low water solubility but also one which does not vary significantly with pH. This permits the pH of the reaction solution to be set using buffers, a pH stat, or the like to optimize enzymatic activity for the particular enzyme chosen.

## Enzymes

Enzymes useful in the practice of the invention are hydrolases categorized in class 3 of the Rules For Classification and Nomenclature of Enzymes (The Nomenclature and Classification of Enzymes, 1972, Elsevier, Amsterdam). The term EC followed by a series of numbers provides the identification of an enzyme pursuant to the recommendations of the Commission on Biochemical Nomenclature. Enzymes useful in the process disclosed herein include hydrolases which act on ester bonds (class 3.1.n.n) and on peptide bonds (class 3.4.n.n). Within class 3.4.n.n, serine proteases are preferred. Thiol and carboxy acid proteases, neutral microbiol metalloproteases, other microbiol proteases, and mammalian peptidases may also be used. In general, any enzyme that is known to cleave ester or amide linkages is a candidate for application in the process of invention to a specific substrate. Preferred enzymes have an E value of at least 5, preferably on the order of 10, in the reaction solution. The enzyme preferably is selected to exhibit optimal activity, selectivity, and stability. The stereospecificity of the enzyme can be such that either a direct or subtractive resolution process may be conducted.

Generally preferred proteases include the alkaline proteases from Aspergillus sp. and Bacillus sp.; potentially useful esterases include porcine liver esterase (E.C. 3.1.1.1.); and potentially useful lipases include, but are not limited to, the lipase from Candida cylindracea (also known as C. rugosa) (E.C. 3.1.1.3).

Other potentially useful proteases include serine proteases from animal sources, such as α-chymotrypsin (E.C. 3.4.4.5) and Trypsin (E.C. 3.4.4.4), both isolated from bovine and swine pancreas; serine proteases from plant sources; carboxy-acid proteases from animal sources, such as Pepsin (E.C. 3.4.4.1), Chymosin (E.C. 3.4.4.3), and Carboxypeptidase A (E.C. 3.4.2.1); serine proteases from microorganisms, generally referred to as subtilisins but isolated from a variety of microorganisms, including naturally occurring species and genetically manipulated species, of such microorganisms as Bacillus subtilis, Bacillus amyloliquefaciens, Bacillus amylosaccharicus and Bacillus licheniformis; serine proteases from Aspergillus sp., such as Aspergillus oryzae and Aspergillus flavus, which may be classified as subtilisins but may also be called Aspergillus alkaline proteases; microbial metallo-neutral proteases isolated from a variety of sources such as Aspergillus oryzae, Bacillus sp. and Streptomyces griseus; and other microbial proteases isolated from sources such as the genera Aspergillus, Bacillus, Mucor, Penicillium, Pseudomonas, Rhizopus, Serratia, Staphylococcus, Streptococcus, Streptomyces and Tritirachium. Proteases isolated from mammalian blood, pancreas, spleen, submaxillary glands and gastro-intestinal mucosa also may be used.

Other potentially useful enzymes include lipases such as those isolated from micro-organisms,

Other potentially useful enzymes include lipases such as those isolated from micro-organisms, such as Pseudomonas aeruginosa, Pseudomonas flourecens, Rhizopus arrhizus, Rhizopus delemar, Rhizopus niveus, Rhizopus oryzae, Rhizopus japonicus, Chromobacterium viscosum, Geotrichium candidum, Aspergillus niger, Aspergillus sojae, Aspergillus oryzae, Mucor miehei, Achromobacter lipolyticum, Alcaligenes sp., Arthrobacter sp. and Candida lipolytica. Pancreatic lipases from various mammalian species and lipase derived from wheat germ may also be employed. Other esterases derived from mammalian sources include, but are not limited to, carboxyl esterase (E.C. 3.1.1.1.), carboxypeptidase A (E.C. 3.4.2.1), acetyl cholinesterase (E.C. 3.1.1.7), pepsin (E.C. 3.4.4.1) and trypsin (E.C. 3.4.4.4). Other microbial sources include Bacillus thermoproteolyticus (or thermolysin), Bacillus amyloliquefaciens and Streptomyces griseus as well as papain (E.C. 3.4.4.10) derived from Papaya sp.

The currently most preferred enzyme for use in the invention as applied to 2-substituted propanoic acid esters is a serine protease derived from Aspergillus oryzae available from Amano Enzyme Corp. under the tradename Prozyme 6.

Depending upon the source, these hydrolase enzymes have a working pH range from about 2 to about 10, with their optimum pH generally falling between 5.0 and 8.5. The temperature range for most enzymes takes place from about 15 to 70° C, with the enzymes usually performing most effectively in the range from about 20-45°C.

In addition to isolated and purified enzymes, it should be noted that the processes of the invention also may be carried out employing relatively impure and/or heterogeneous enzyme preparations, such as those derived from cell extracts, cell lysates, partially purified enzyme isolates and whole cells, albeit at some reduction in the enzymatic activity. Enzyme can also be immobilized on solid supports by conventional means such as covalent binding, adsorption, matrix entrapment, ion-exchange, or microencapsulation. Indeed, enzymes contained within whole cells, whether viable or not, may also be used in the practice of this invention, and accordingly, the term "enzyme" as used herein is meant to broadly include biocatalytic enzymes in all of these forms. Of course not every enzyme will perform optimally on every substrate, but those skilled in the art will be well aware of how to select and test the activity of any particular enzyme in any particular system with routine and simple experimentation in view of this disclosure.

## Process Parameters and Design Considerations

Broadly, in operation of the process of the invention, the water soluble reactant ester (e.g., stereoisomers in racemic mixture) is supplied to the reactor in an aqueous phase containing donor alcohol and having a pH suitable for enzyme activity. The dissolved reactant encounters the enzyme which stereoselectively catalyzes its transesterification to a product ester which has preferably significantly reduced solubility in water (or, conversely, increased solubility in organic solvents). The product species accordingly can partition spontaneously to form a water immiscible organic phase if its melting point is below the reaction temperature, may precipitate

as a solid material if its melting point is above the reaction temperature, or may be absorbed into a water-immiscible organic solvent across a phase boundary. Depending upon the chiral character of the racemic mixture and the identity of the enzyme used, the product ester may contain either optically pure compounds or mixtures substantially enriched in particular isomeric forms, i.e., the (S) form or the (R) form.

To optimize the resolution process, high partition coefficients between reactant and product esters are preferred. Under ideal circumstances partition coefficient ratios can exceed 20 or more leading to very efficient reactor operation and reactant/product stereoisomer separation. However, extreme solubility differences are not required in the practice of the invention.

The organic extractant phase, if one is used as preferred, preferably is a solvent that is miscible with water to an amount less than 3%, and which minimizes the extraction of donor alcohol from the aqueous phase containing the enzyme.
Furthermore, since the transesterification reaction typically is accompanied at least to some extent by enzymatic hydrolysis to produce the acid form of the product ester, the organic phase should be chosen so as to dissolve both the transesterification product and the enzyme catalyzed hydrolysis product.
Nonlimiting examples of solvents that may be used for extraction of product esters include aliphatic and aromatic hydrocarbons such as hexane, cyclohexane, xylenes, and toluene; chlorinated solvents such as methylene chloride and chloroform; water-immiscible alcohols including octanol and decanol; esters including amyl acetate and butyl acetate; ethers such as tertiary butyl methyl ether; and ketones such as methyl isobutyl ketone.
In choosing a solvent for use in the extractive reactor processes, important considerations include its compatibility with the enzyme, its toxicity, viscosity, solubility, and miscibility characteristics as well as its ability to be separated readily from other reaction components and its inertness toward materials used in the reactor design including any membrane used. The currently preferred solvent for use in extracting NSAID reaction products is toluene.

In the process of the invention the aqueous phase containing the enzyme preferably is held at a pH value amenable to the enzyme in use. This may be accomplished, for instance, by buffering or using a pH stat. Typical ideal pHs are neutral to slightly alkaline. In general, the preferred pH may range between about pH 5 and pH 9, but some enzymes which may be useful in specific sparation processes are known to have extreme pH optima. By adjusting the pH with a pH stat, one may employ nonbuffering salts to adjust the ionic strength of the aqueous phase to a level desirable for maintaining the activity, selectivity, and stability of the chosen enzyme. Further the ionic strength of the aqueous phase may also be chosen to be sufficiently high so as to aid in the partition of hydrolysis product (i.e., the product of chemical, nonspecific hydrolysis, if any, and any enzymatic hydrolysis) from the aqueous into the organic phase. A buffer system may be used which is appropriate for maintaining the activity, selectivity, and stability of the chosen enzyme and which does not unduly restrict the solubility of the reactant ester. Well known sodium phosphate buffer systems may be used to advantage, typically at concentrations between 0.05M and 1M.

The temperature preferably is held between about 15°C and 30°C, most preferably 22°C. Generally, the temperature is selected so as to keep all species in solution and to permit optimal enzymatic activity.

The donor alcohol present in the aqueous phase preferably is water soluble and most preferably is miscible in all proportions with water, or at least to a concentration which permits obtaining the maximum ratio of transesterification to hydrolysis. In preferred embodiments, ethyl alcohol is used at a concentration ranging generally between about 2M and 5M (i.e., 8% v/v and 20% v/v, respectively). In the currently most preferred case, ethanol is the alcohol of choice and is present in the initial concentration of about 2.5M (10% v/v).

In these various process methods, once the enzymatic reaction has taken place, the aqueous stream contains the enzyme as well as unreacted stereoisomer. Typically, systems for implementing. the method will include means for an enzyme/unconverted isomer separation. The dissolved enzyme may be removed from the isomer solution by known techniques such as ultrafiltration, diafiltration, dialysis, precipitation, or adsorption. Alternatively, the enzyme may be immobilized on a support which is contained in a reaction vessel (e.g., a packed- or fluidized-bed) or which is separable from the aqueous isomer solution by techniques such as filtration, microfiltration, ultrafiltration or centrifugation.

The unconverted reactant ester often also can be separated from the enzyme by altering the isomer's water solubility by chemical or environmental change (e.g., adding salts, reducing the temperature, or adjusting the pH). Chemical conversion of the enzyme-inactive reactant ester to produce the corresponding chiral acid can be effected by a non-enzymatic hydrolysis reaction which takes place at a temperature from about 20 to 80°C and a pH ranging from about 10 to about 14. Once the separation is completed, the enzyme can be recycled to the reactor vessel and the isolated reactant can be further processed.

A second aqueous phase may be used to extract the product ester and any acid form contained in the organic phase. This second aqueous phase preferably is held at a specific pH level such that the parent carboxylic acid of the reactant ester is easily extracted and held in the aqueous phase as the carboxylate anion.

The pH of the extracting aqueous phase is controlled preferably by a pH stat. A typical composition for this aqueous extractant is 0.1M sodium carbonate maintained at pH 10 by a pH stat equipped to deliver a concentrated aqueous solution of sodium hydroxide.

Details of flow configuration can affect a number of secondary aspects of extractive reactor process performance including product purity, reactant conversion, phase separation and pH control. For the preferred form of reactor involving extraction across a membrane as described below, reactants enter and products leave the enzyme-activated membrane by diffusive processes in response to their local concentration gradients and consistent with their aqueous/organic partitioning behavior.

Thus, the flowrate of the feed stream (if any) preferably is adjusted so as to obtain the desired degree of enzymatic conversion of the key reactant. The flowrate of the organic stream on the opposite side of the membrane preferably is adjusted to permit the organic-soluble enzymatic reaction products to be withdrawn at an appropriate concentration. In the preferred extractive membrane reactor system, it may be desirable to flow the organic process stream past the membrane at a relatively high rate. Operation in this manner facilitates prompt and efficient removal of product from the enzymatic reaction zone, since high organic stream flowrates translate to low product concentrations, and large concentration gradients.

In this way, the yield and productivity of the enzymatic reaction may be improved, as reaction equilibrium is biased further toward effective transesterification.

The relationship between the flowrates of the organic and aqueous process streams can also effect stereochemical purity of the products removed from the process. The flows of the aqueous and organic process streams may be either cocurrent or countercurrent, and the extractive reactor process may be operated either batchwise (with optional recycle of one or both process streams), in a continuous mode, or in a semi-batch fashion.

Additional processing steps (e.g., repeated application of the resolution process, recycling of the racemic mixture fluid, or purification of unconverted reactants and/or products) may improve the efficiency of the resolution process, and the chemical and stereochemical purity of the chiral products produced therein. For instance, where an optically resolved product exits an extractive reactor in an organic solution, this organic process stream may be pumped to a holding tank and treated with a basic solution in order to remove the acid from the organic solution, with optional organic solvent recyle. Alternatively, the product may be separated from the organic fluid and purified by evaporation of the volatile organic solvent and attendant crystallization of the resolved product. In addition, the chiral product can be racemized, converted back to the chemical form of the starting racemic stereoisomer mixture, and reintroduced to the membrane. When an optically purified organic-soluble product exists in the form of a neat organic liquid or as a solution in a volatile organic solvent, the product may be further purified by distillation or by solvent evaporation.

In operational aspects, the resolution process is initiated by the addition of enzyme to the aqueous phase containing the substrate and donor alcohol. The process is then allowed to proceed undisturbed until a sufficient degree of enzymatic conversion of the aqueous soluble substrate ester has been achieved. The enzymatic reactions are then terminated, e.g., by lowering the pH to approximately 4, e.g., with sulfuric acid. The extractive processes are then allowed to proceed undisturbed (if not previously completed during the course of the reaction) until substantially all the parent carboxylic acid and product ester have been extracted from the aqueous phase into the organic phase, and then (optionally) to the high pH extracting second aqueous phase. The cessation of addition of sodium hydroxide titrant to the extracting aqueous phase by the pH stat can be a useful indicator that the process is complete. The product ester may be recovered from the organic phase and the acid form recovered from the aqueous phase by conventional methods.

Finally, enantiomerically enriched forms of the parent carboxylic acid may be obtained from the aqueous reaction solution by chemical hydrolysis of the remaining substrate ester. This may be accomplished by hydrolyzing the remaining substrate reactant ester under basic conditions, e.g., raising the pH of the aqueous solution to about 12.5 by addition of sodium hydroxide, and maintaining the pH at this value for 1-18 hours. The pH is then brought to approximately 3, and the liberated carboxylic acid extracted by conventional means, preferably using an extractant from which the desired carboxylic acid may later be recrystallized. Acidic hydrolysis also may be used.

## Membrane Reactor Systems

The process of the invention is unlimited with respect to the types of reactor designs which it can exploit. However, as disclosed more fully below, the preferred process configuration of the invention is extractive, that is, a process in which the desired product is partitioned into the organic phase leaving the unwanted enantiomer behind. More particularly, the process preferably is implemented in an extractive membrane reactor of the type generally disclosed in copending U.S. Application Serial No. 07/178,735. The hydrolase enzyme in such

designs may be disposed in solution in the aqueous phase, immobilized on the surface of the membrane, or both. Apparatus designed to exploit direct resolution processes (c.f. subtractive processes) also may be used, but, practically speaking, direct resolution processes require an enzyme with a high E value. The invention accordingly may be practiced in this way, but there are fewer suitable enzymes available.

Enzyme-activated membranes suitable for the process are chosen with consideration of the chemistry of the membrane, its morphology and the mode of enzyme activation.

The membrane material should be such that it will not be deleteriously effected (e.g., swollen or chemically attacked) by any of the components of the reactor system and, in particular, by reactants, products, or solvents. Although membranes comprising inorganic materials (e.g., ceramics) can be used in the practice of this invention, polymeric membranes represent a preferred embodiment. In particular, membranes fashioned from solvent-resistant polymers are preferred. Typical polymeric materials that can be fabricated into membranes suitable for the practice of this invention include regenerated cellulose, the esters of cellulose (and the particularly preferred partial acetate and nitrate esters), polyacrylonitrile and copolymers thereof (especially the hydrophilic ones including copolymers incorporating acrylamide, acrylate, acrylate esters and/or methacrylate functionalities), polyurethane-containing copolymers; the polyarylsulfones and polyarylethersulfones (including polysulfone, polyethersulfone and blends thereof with such hydrophilic copolymers as polyethyleneoxide, polyhydroxyethers, polyethyleneglycol, polyvinylalcohol, polycaprolactone and polyepihalohydrins), polyvinylidene fluoride, polytetrafluoroethylene, polyvinylalcohol, aliphatic and aromatic polyamides, polyimides, polyetherimides, polyesters, polycarbonates, polyolefins such as polypropylene and polyvinylchloride, polybenzimidazole and polybenzimidazolone.

It is preferred that the membrane be water-wet or hydrophilic, particularly if enzymatic reaction is to occur within the membrane. Certain of the above membrane polymers are intrinsically hydrophilic (e.g., the cellulosics, many polyacrylonitrile copolymers and the polyamides). However, even those which are not intrinsically hydrophilic may be modified by an appropriate chemical or physical surface treatment (e.g., by derivatization or attachment of hydrophilic functional groups or simply by contact with an appropriate surfactant), by coating the pore wall surfaces of hydrophobic polymers with hydrophilic materials, or simply by filling the pore volume of an asymmetric, microporous membrane with hydrophilic enzyme in the form of a high-water-content gel.

Membranes suitable for use as enzyme supports can exhibit various morphologies. In particular, they may be microporous membranes of the type generally employed in microfiltration processes, wherein the pore sizes will typically range from a few hundredths of a micron to several microns, and pore void volumes range from a few percent to 90% and greater. Such microporous membranes may be isotropic (i.e., with no significant variation in pore size from one external surface to the other), or they may exhibit some degree of anisotropy. Ultrafiltration membranes are also useful in the practice of the method. They are typically highly asymmetric, and characterized by a very thin skin with effective pore sizes in the range of approximately from 1 to 20 nm residing atop a much thicker but more highly porous substrate region comprised of much larger pores. Additionally, gel-type dialysis membranes (e.g., regenerated cellulose membranes of the type used in hemodialysis) may also be employed, particularly where the enzyme is located at the surface of the membrane. Such membranes may be surface-activated either by covalent attachment of the enzyme to an exterior surface or by formation of a dynamic enzyme gel-polarized "secondary membrane" layer at one surface. In addition, the membrane is largely microporous, characterized by a relatively thick, high-void volume, finely porous spongy substrate region, but it possesses an ultrafiltration-type skin at one external surface.

Such membrane morphologies offer high enzyme loadings and facilitate periodic enzyme replacement. The use of skinned microfiltration membranes with this morphology is described below in connection with modes of activation of membranes with enzymes.

The geometry of the membrane employed in the extractive membrane reactor embodiment of this novel method is a secondary consideration, and membranes in the forms of flat sheets, tubes preferably of large-diameter, and hollow fibers of various diameters are all useful. Membrane module comprising bundles of hollow fibers, or a spirally wound surface, and two inlet ports and two outlet ports are preferred. Plate-and-frame or cassette-type housings are preferred for packaging flat-sheet membrane over spiral-wound cartridges, whereas tubular and hollow-fiber membranes are packaged efficiently in cylindrical multitube or multifiber membrane modules, the construction of which resembles that of a shell-and-tube exchanger. Membranes in the form of hollow-fiber modules permit large areas of membrane to be packaged tightly and economically.

By virtue of the containment, entrapment, or immobilization of enzyme within the membrane phase, any leakage of enzyme out of the membrane and into either the aqueous or organic process streams may be substantially prevented. Suitable means of enzyme incorporation include containment within the pore spaces of asymmetric (i.e., skinned), microporous membrane structures, adsorption on membrane pore wall surfaces, encapsulation or entrapment in polymeric gels within membrane pores, covalent coupling to membrane pore

walls, and crosslinking of enzyme, either within the pore spaces or adsorbed on membrane pore wall surfaces. In addition, the enzyme can be immobilized on a particulate, solid-phase support and that immobilized enzyme can be contacted with the aqueous fluid.

A number of alternatives exist for the activation of membranes with enzymes. The most straightforward approach involves covalently linking the enzyme to the exterior or interior (i.e., pore wall) surfaces of membranes via any of a number of conventional enzyme immobilization chemistries developed for attaching biocatalysts to non-membrane supports. [See: Zaborsky, O.R., Immobilized Enzymes, CRC Press, Cleveland, OH (1973) ; Weetal, H.H., Immobilized Enzymes, Antigens, Antibodies, and Peptides: Enzymology, Vol.1, Marcel Dekker, N.Y. (1975); Emery, A. et al., Biotechnol. Bioeng., 16:1359 (1974)]. Enzymes also may be crosslinked in porous membranes (Thomas, D. and S.R. Caplan, pp. 351-398 in Membrane Separation Processes, P. Meares, ed., Elsevier, Amsterdam, 1976), entrapped in membrane gels (Blaedel, W.J. et al., Anal. Chem., 44:2030, 1972), encapsulated, or adsorbed on or within membranes via ion-exchange or other specific or non-specific protein-surface interactions. It is also forseeable that in the practice of this method it will prove effective in some cases to combine the above techniques. For instance, enzyme may first be adsorbed on membrane exterior or pore wall surfaces, subsequently to be anchored more positively by crosslinking the adsorbed enzyme layer in place.

In addition, the enzyme may be reversibly contained in a skinned, microporous membrane as described in the U.S. Patent No. 4,795,704 issued January 3, 1989, which is incorporated herein by reference. Such a membrane structure is capable of entrapping the enzyme between two boundaries not traversable under normal membrane reactor operating conditions. These two barriers consist of (1) the "skin" or surface layer of the enzyme-activated membrane, which contains pores that are sufficiently small so as to prevent the transport and leakage of macromolecular enzyme from the porous interior of the membrane to the aqueous process stream in contact with it, and (2) the aqueous/organic phase boundary at the opposite membrane surface which, by virtue of the insolubility of most enzymes in organic solvents, prevents the enzyme from partitioning into the hydrophobic phase and thus escaping from the membrane via the organic process stream. Such a membrane may be activated with enzyme by ultrafiltering an aqueous solution of enzyme through it, with very high loadings of active enzyme being incorporated in this manner. An additional benefit of this mode of enzyme activation is its reversibility. That is, deactivated enzyme may simply be removed from the membrane by a back-flushing operation, thus facilitating the periodic replenishment of the enzyme catalyst.

An alternative preferred approached to enzyme activation of membranes relies on the formation of dynamic enzyme-gel-polarized membranes atop the surface of ultrafiltration or gel-type membranes of the type used, for example, in hemodialysis and hemofiltration.

Dialysis/ultrafiltration membranes comprising regenerated cellulose or hydrophilic polyacrylonitrile-based polymers and copolymers are particularly preferred. Although such membranes are finely porous and water-permeable, the effective surface pore size of membranes suitable for use here typically are too small to admit enzymes. However, it has been demonstrated [Kerkhof, P.I.A.M., et al., "Enzymatic Hydrolysis of Lipids in a Membrane Reactor," a poster presented at the International Membrane Technology Symposium, Lund, Sweden, May 28-30, 1985; Molinari, R. and E. Drioli, Proc. Nat. Congr. Ind. Chem. Div. Sci., Siena, June 10-25, 1985] that such membranes can be coated effectively with enzymes by the simple expedient of ultrafiltering an aqueous enzyme solution across the membrane, thus leaving a concentrated enzyme gel layer deposited on one surface of the membrane film or fiber. If the organic process stream is subsequently brought into contact with the surface of the membrane so activated, the enzyme gel layer will tend to remain in place at the membrane surface, inasmuch as the enzyme is not appreciably soluble in organic solvents. In a refinement of this technique, the enzyme surface layer may be stabilized by chemically crosslinking the enzyme protein.

Resolution may also be effected within the aqueous phase containing dissolved enzyme. The water-wet portion of the membrane is accessible to the enzyme solution in this embodiment. A microporous membrane with a fine spongy matrix or a high-molecular-weight-cutoff ultrafiltration membrane (i.e., with a cutoff higher than the enzyme molecular weight or size) will allow the enzyme to enter the membrane along with the aqueous fluid. Enzymatic reaction will occur in the aqueous fluid that is circulating past the membrane surface (i.e., fluid not within the membrane structure) and in the aqueous solution present within the membrane interior (i.e., fluid associated with the membrane).

Where the enzyme is dissolved in the reactant solution, the enzyme if desired may be excluded from entering the membrane by, for example, size exclusion. A tight, gel-type, dialysis membrane, e.g., regenerated cellulose, will reject macromolecules such as proteins and enzymes. A low molecular weight cutoff, ultrafiltration membrane (i.e., with a cutoff lower than the enzyme molecular weight or size) will also exclude the enzyme from the interior of the membrane.

Finally, another mode of action involves use of a hydrophobic membrane which is preferentially wet by the organic phase. The enzyme is excluded from the membrane interior by virtue of the low solubility of enzymes

in most organic solvents. An organic-wet membrane must be operated, such that a small aqueous-to-organic pressure difference across the membrane is maintained at all times. This pressure differential serves to prevent ultrafiltrative flow of the organic phase through the membrane. Similarly, the hydrophilic membranes of the type discussed above are exploited with maintenance of a small organic-to-aqueous pressure differential.

## Preferred Reactor Structure

The sole figure of the drawing illustrates schematically a currently preferred form of reactor design useful in the practice of the invention as set forth above. It comprises two modules 10, 20, respectively, which house a high surface area membrane 22, 22', respectively, of a nature and disposed in a configuration as set forth above. A reactant ester feed stream 12, containing the donor alcohol and optionally also the hydrolase enzyme, enters reactor 10, contacts a surface of membrane 22, and exits reactor 10 as an at least partially depleted stream 14. The other side of membrane 22 of reactor 10 is exposed to an organic extractant which enters through inlet 16 and exits through port 18 to communicate with the second module 20. The organic phase enters module 20 at inlet 28, contacts one surface of the membrane 22' contained in the module, and is sent back to module 10 via exit port 26. Disposed with the line connecting organic extractant exit 26 of module 20 and inlet 16 of module 10 is an optional product ester recovery unit 30. Unit 30 can take many forms as will be apparent from the foregoing. Its function is to recover the product ester and optionally to reconstitute the organic extractant for reuse by delivery to inlet 16.

Module 20 is serviced by an aqueous extraction solution input line 22 which contacts the surface of membrane 22' opposite that of the organic extractant and then exits the module at least partially loaded via stream 24.

In operation, the product ester is extracted across membrane 22 in module 10 as hydrolase enzyme entrapped in the membrane, immobilized in or on the membrane, or simply dissolved within the reactant solution, converts the water soluble reactant ester to a water insoluble product ester stereoselectively by transesterification. Unreacted reactant ester exits through line 14. Transester product, together with other oil soluble components such as the acid form of the reactant ester produced in the reaction solution either enzymatically or chemically, is absorbed into the organic loop across membrane 22. The product loaded organic stream passes through line 19, entering module 20. There, any dissolved acid form product is extracted across the membrane 22' into the aqueous extractant solution having a composition designed to optimize solubility of the acid form as discussed above. The aqueous extractant 24 exits module 20 for further processing for recovery or recycle of the enantiomerically enriched acid form, if any. Organic phase containing the product ester exits module 20 for recirculation via inlet 16 or for product recovery in module 30.

The invention will be understood further from the following non-limiting examples.

Preparation of 4-sulphomethyl ester of 2-(4-isobutylphenyl) propanoic acid (racemic, sulphomethyl ester of Ibuprofen).

One hundred three grams (0.5 mol) of racemic 2-(4-isobutylphenyl) propanoic acid are mixed with 67 grams (0.5 mol) sodium formaldehyde bisulfate in a beaker containing 125ml trifluoroacetic acid. The mixture is stirred as trifluoroacetic acid anhydride (105g, 0.5mol) is added. The mixture is warmed as the reactants dissolve, and after 30 minutes the hazy solution is filtered through a glass frit. After the filtrate is evaporated under vacuum, the residue is dissolved in water, and the small amount of unreacted racemic 2-(4-isobutylphenyl) propanoic acid which precipitates is removed by extraction with hexane. The aqueous filtrates are then saturated with sodium chloride causing the product to separate as a white solid. This material may be isolated by filtration and dried to produce 130 grams of the water soluble Ibuprofen sulphomethyl ester at 81% yield. The compound may be partially purified by dissolving in water and reprecipatating with addition of sodium chloride. The compound has the structure:

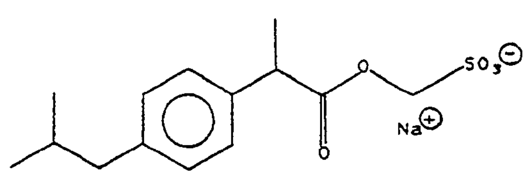

Formation of Ibuprofen Methyl Ester by Transesterification in Methanol Solution

Examples 1-4

10 mmols of racemic Ibuprofen sulphomethyl ester were dissolved in water containing 200mM sodium phosphate buffer, pH 7, and varying amounts of methanol. Buffer volume was varied so that the total reaction volume was 100ml. To the resulting aqueous alcholic substrate solution was added a protease derived from Aspergillus oryzae (Prozyme 6, Amano Enzyme Corporation, 60,000 units per gram solid).

The resulting enzymatic reaction solution was incubated at ambient temperatures (22-23°C) for 18 hours. After this time, the reaction mixture was acidified, and all organic soluble material removed from the reaction by extraction with three volumes of diethyl ether. The combined organic layers were dried over magnesium sulfate, and evaporated under reduced pressure to leave a viscous oil. Thin layer chromatography analysis of the product on silica gel, together with an authentic sample of Ibuprofen methyl ester used as a standard, indicated that both Ibuprofen acid form and Ibuprofen methyl ester were formed in the enzymatic reactions.

The oil was examined using Fourier transform infrared spectroscopy. Absorbances at 1709cm$^{-1}$ and 1740cm$^{-1}$ indicated the presence of both acid and methyl ester forms of Ibuprofen. The ratio of the absorbances of the acid carbonyl function at 1709cm$^{-1}$ to the ester carbonyl function at 1740cm$^{-1}$ was calculated, and taken as a indication of the relative amount of methyl ester formed via transesterification versus acid formed via enzymatic and/or chemical hydrolysis. The data is set forth below in Table 1.

Table 1

| Example | Methanol Concentration (M) | 1740/1709 |
|---|---|---|
| A | 0 | 0 |
| B | 0.2 | 0.68 |
| C | 2.0 | 3.7 |
| D | 5.0 | 8.9 |

As indicated by the data, in the absence of a donor alcohol (A), no transesterification occurs. In the presence of 0.2 M methanol, the ratio of methyl ester to acid form is approximately 1-3. At 2 M methanol and 5 M methanol, transesterification predominates over hydrolysis.

Examples 5-8

25 mmol of racemic Ibuprofen sulphomethyl ester were dissolved in 100 ml of 500 mM sodium phosphate buffer, pH 7, containing 1.0M of various water soluble alcohols. To this solution was added Prozyme 6 (100 mg) and the reaction solution was allowed to incubate at ambient temperature (22-23°C) for 18 hours. After this time, the reaction mixture was acidified to hydrolyze remaining sulphomethyl ester to acid form, and all organic soluble material was removed by extractions with three volumes of diethyl ether. The combined organic layers were again dried over magnesium sulfate and evaporated under reduced pressure. Spectroscopy of the viscous oil which resulted compared the absorbances of the Ibuprofen acid carbonyl peak at 1709cm$^{-1}$ with the ester carbonyl peak at 1735-40cm$^{-1}$. The results are set forth in Table 2.

Table 2

| Example | Alcohol | Absorbance (1709) | Absorbance (1735) |
|---|---|---|---|
| 5 | MeOH | 0.59 | 1.00 |
| 6 | EtOH | 0.50 | 0.75 |
| 7 | $^{n}$PrOH | unobservable | 0.50 |
| 8 | $^{i}$PrOH | 1.00 | unobservable |
| 9 | $^{n}$BuOH | 0.20 | 0.22 |

As indicated by the data, with the exception of isopropyl alcohol, all of the water soluble alcohols tested were operative as donor alcohols in this transesterification reaction, even at the relatively low 1.0 M concentration used.

Formation of Enantiomerically Enriched Naproxen Ethyl Ester by Transesterification of Racemic Naproxen Choline Ester

The methyl sulfate salt of Naproxen choline ester (choline ester of 2-(6-methoxy-2-naphthyl) propanoic acid) may be prepared as follows. 23 grams (0.1 mol) of racemic Naproxen (2-(6-methoxy-2-naphthyl) propanoic acid) are stirred in a solution of 200 ml dichloromethane, 7.3ml (0.1 mol) thionyl chloride, and 5 drops dimethyl formamide. The mixture is brought to reflux for one hour, during which time the Naproxen acid dissolves, and HCl and sulfur dioxide are generated. Heat is then removed and the reaction mixture allowed to stir overnight at ambient temperature. The dichloromethane is removed on a rotary evaporator to leave a viscous yellow oil. This oil is dissolved in 50ml tetrahydrofurane, and added dropwise to a solution of 2-(N,N-dimethyl amino) ethanol (2.0 equivalents, 18.0ml) in 100 ml THF, the reaction flask being cooled in an ice bath. Addition is complete in 20 minutes, and the reaction is allowed to stir an additional hour at ambient temperature. The THF is removed on the rotary evaporator and the viscous residue poured into IM NaOH and extracted with diethyl ether (3 x 200ml). The combined organic layers are washed with water (2 x 300ml), and dried over potassium carbonate. Evaporation leaves the N, N-dimethyl ethanol amine ester as a pale yellow oil (20.0g recovered, 66% yield). The ester is dissolved in a 100ml of propanol to which is added 6.2ml dimethyl sulfate. The reaction mixture is brought to reflux for 15 minutes, cooled to ambient temperature, and the alcohol removed on a rotary evaporator to leave the quarternary ammonium salt as a viscous pale yellow oil, which slowly solidifies. This product may be recrystallized from acetone to leave a white, solid material. The structural formula of the product is:

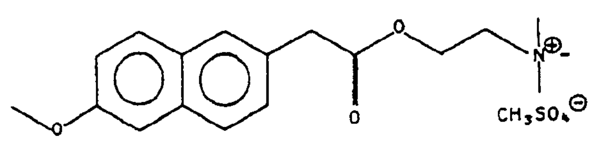

Examples 10,11, and 12

10mM of the Naproxen choline ester were dissolved in 100 ml of 200mM sodium phosphate buffer at varying pH values, which had been made 2.5M in ethanol. To the solution was added Prozyme 6 (100mg), and the reaction was allowed to proceed at ambient temperatute for 18 hours. After this time, the reaction mixture was made basic to approximately pH 9 by the addition of sodium hydroxide and then extracted 3 times with an equal volume of diethyl ether. The combined organic layers were dried over magnesium sulfate and evaporated under reduced pressure to leave a solid. Thin layer chromatography on silica gel, Fourier transformation infrared spectroscopy (FTIR), and high pressure liquid chromatography analysis on a Chiracel OK column, (using a mobile phase of 9:1 hexane:isopropyl alcohol and authentic standards) indicated that this material was Naproxen ethyl ester enriched in the (R)-enantiomer. The results of these experiments are set forth in Table 3 below. In the

table, the "% conversion" refers to the percent of the total starting substrate material converted to ethyl ester. The α values for the recovered ethyl ester were measured in chloroform, and the E value is calculated according to Chen et al., J.A.C.S., 104, 7294, 1982.

Table 3

| Example | pH | %conv. | α[1] | %ee[2] | E[3] |
|---|---|---|---|---|---|
| 10 | 5.0 | 4.7 | -0.374° | 77.4 | 8.2 |
| 11 | 6.0 | 33.1 | -0.356° | 73.7 | 9.4 |
| 12 | 7.0 | 54.3 | -0.299° | 61.9 | 9.1 |

1. Optical rotation as measured in a polarimeter.
2. Calculated using $[\alpha]_D$ at 66.0° at C = 1 in $CHCl_3$.
3. E is equal to $(V_R K_{MR})/(V_S K_{MS})$, where V is the reaction velocity, and $K_M$ is the Michaelis constant.

These data show that the hydrolase enzyme Prozyme 6 transesterifies selectively the (R) enantiomer of the Naproxen choline ester at a rate almost and order of magnitude greater than the (S) enantiomer. Enzymatic activity is optimized at neutral pH.

Synthesis or Racemic Naproxen Ethyl Sulfate Ester

Racemic Naproxen acid (50mmol, 11.5g) was stirred in 200ml of isopropyl alcohol. To this was added potassium tertiary butoxide (50mmol, 6.11g). The reaction mixture rapidly became thick and sufficient methanol was added to return the mixture to an almost clear solution. To this solution was added 1.0 equivalents (6.2 g) of cyclic ethyl sulfate, prepared by the method of Gao and Sharpless (J.A.C.S., 110, 7538, 1988). The resulting reaction mix was exothermic, and quickly solidified. The entire reaction mixture was put into a 500 ml volume of diethyl ether, and the solid material was removed by filtration and dried under vacuum. Infrared spectroscopy of the resulting material showed a carbonyl absorbance at 1735cm$^{-1}$, no acid carbonyl absorbance band, and was otherwise consistent with the structure of Naproxen ethyl sulfate ester having a structure as set forth below.

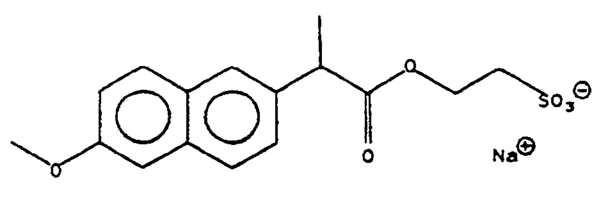

Examples 13, 14, 15, 16

10mmol (3.76g) of the racemic ethyl sulfate ester were dissolved in 100 mls of aqueous reaction media composed of 200 mM sodium phosphate buffer at various pH values, and made 2.5M in either methanol or ethanol. To this solution was added Prozyme 6 (100 mg), and the reaction solution was allowed to incubate at 25°C for 18 hours. After this time, the reaction mixture was made basic to approximately pH 9 by the addition of sodium hydroxide, and then extracted three times with an equal volume of diethyl ether. The combined organic layers were dried over magnesium sulfate and evaporated under reduced pressure to leave a solid material. FT/IR spectroscopy and a high pressure liquid chromatography analysis (using a mobile phase of 9:1 hexane: isopropyl alcohol and authentic standard samples) indicated that the product materials were Naproxen methyl and ethyl esters variously enriched in the (R)-enantiomer. The results of these experiments are tabulated below in Table 4. In the table, the "percent conversion" refers to the percent of the total starting substrate material converted to methyl or ethyl ester. The α values for the recovered esters were measured in chloroform

([α]$_D$ for (R)-Naproxen ethyl ester = -71.8°, [α]$_D$ for (R)-Naproxen methyl ethyl ester = -48.3°, both in chloroform at C = 1.0), and the E value is calculated according to Chen et al, supra.

Table 4

| Example | pH | alcohol | %conv. | α | %ee | E |
|---|---|---|---|---|---|---|
| 13 | 6.0 | MeOH | 12.3 | -0.692° | 96.4 | 62.3 |
| 14 | 6.0 | EtOH | 3.9 | -0.427° | 92.2 | 25.6 |
| 15 | 7.0 | MeOH | 18.0 | -0.601° | 83.7 | 13.5 |
| 16 | 7.0 | EtOH | 18.1 | -0.435° | 94.0 | 39.6 |

These data indicate that highly enantiospecific transesterification has occurred.

Example 17

25.8g (0.1 mol) of enantiomerically pure Naproxen ethyl ester were dissolved in 30 mls of 95% ethanol at reflux. To this was added 4.4g (0.11 mol) sodium hydroxide followed by 5 ml water. The resulting reaction mixture was heated at reflux for one hour, after which time HPLC analysis of the reaction mixture on a Chiracel OK column using a mobile phase of 9:1 hexanes: isopropyl alcohol showed complete racemization and hydrolysis of the ethyl ester. The resulting solution was cooled and the pH adjusted to approximately 6.5 by the addition of racemic Naproxen acid (2.5g, 0.011 mol). This racemic mixture, essentially insoluble in water, was converted to water soluble ethyl sulfate ester as follows. Ethylene sulfate (12.4g, 0.1 mol) was added incrementally at a rate sufficient to keep the reaction mixture near reflux. The total addition of ethyl sulfate took approximately four minute, after which the reaction mixture became a solid mass. This product was dissolved immediately in 200 ml of water and 50 ml of 1.0 M sodium phosphate buffer, pH 6.5. The resulting solution was adjusted to pH 7.0 with 50% aqueous sodium hydroxide, to give a total solution volume of 315 ml. HPLC analysis of this solution using the conditions described above indicated that 6.5g (0.028 mol) racemic Naproxen acid were present in the total solution, with 31.2g (0.083 mol) racemic Naproxen ethyl sulfate ester. This indicated that the ester formation had proceeded to 83% completion. Total concentrations of all species in the solution accordingly were:

| | |
|---|---|
| ethanol | 10% |
| sodium phosphate | 0.350 M |
| racemic Naproxen acid | 0.089 M |
| racemic Naproxen ethyl sulfate ester. | 0.263 M |

To this solution was added 5.7 g of the commercial preparation of Prozyme 6. The resulting reaction mixture was incubated at ambient temperature for 17 hours, after which the reaction mixture had become a solid mass. The mass was broken up by vigorous stirring, and the resulting slurry acidified to approximately pH 3 by the careful addition of concentrated HCl. The resulting slurry was extracted three time with equal volumes of a 1:1 mixture of heptane and diethyl ether. The combined organic layer was then back-extracted five times with a 10% aqueous solution of potassium carbonate. The remaining organic layer was dried over magnesium sulfate, and evaporated under reduced pressure to leave 8.5g of Naproxen ethyl ester. HPLC analysis showed the material to be the (R)-enantiomer, and to be present with an enantiomeric excess of 86%. Based on the initial quantity of racemic Naproxen ethyl sulfate ester, this represented a transesterification of 39.6% of the total substrate.

The potassium carbonate washings were combined and acidified, precipitating 3.3g of Naproxen acid. HPLC analysis revealed this Naproxen acid material to be present with (R)-enantiomeric excess of 26.3%. Of course, this material included the racemic Naproxen acid which was not esterified by the ethyl sulfate reagent, and the racemic Naproxen acid added to lower the pH of the racemization/hydrolysis reaction.

The aqueous layer remaining from the enzymatic reaction mixture was analyzed to reveal the degree of enantiomeric enrichment which had occurred. 10% of the remaining aqueous layer was acidified with concentrated HCl to approximately pH 1, and heated to boiling. A Dean-Stark trap was used to remove the resisdual ethanol present. After 30 minutes, the reaction mixture was cooled, and the precipitated Naproxen acid recovered by filtration. HPLC analysis indicated this material was present with an enantiomeric excess of 68.9% of the (S)-enantiomer. 1.0g of Naproxen acid was recovered, indicating that 16.3g (0.043 mol) of Naproxen ethyl sulfate ester with an enantiomeric excess of 68.9% of the (S)-enantiomer were present in the original aqueous enzymatic reaction mixture when the reaction was terminated.

This example illustrates a process in which an enantiomerically enriched Naproxen ethyl ester is chemically transformed into a racemic Naproxen ethyl sulfate ester, and then treated in accordance with a process of the invention by transesterification to produce enantiomerically enriched (R)-Naproxen ethyl ester and enantiomerically enriched (S)-Naproxen acid. The recovered (R) Naproxen ethyl ester of course may be recycled, permitting a theoretical use of 100% of the Naproxen starting material.

<u>Examples 18, 19, 20</u>

10 mmols of Ketoprofen choline ester (methyl sulfate salt) having the structure set forth below were dissolved in 100 ml of 200 mmols sodium phosphate buffer at pH 7 which had been made 2.5M in ethanol.

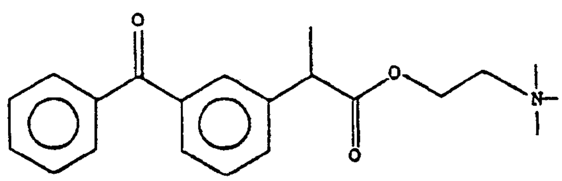

$CH_3SO_4^-$

To this solution was added 100 mgs of various commercially available proteolytic enzyme preparations. (Prozyme 6 described above, Protease 8, and Protease 27, both derived from Bacillus subtilis, and supplied by Sigma Chemical Company). The reaction solution was allowed to incubate at 25°C for 18 hours. After this time, the reaction mixture was made basic to approximately pH 9 by the addition of sodium hydroxide, and then extracted three times with an equal volume of diethyl ether. The combined organic layers were dried over magnesium sulfate and evaporated under reduced pressure to leave a solid material. FT/IR spectroscopy gave results consistent with the product being ethyl ester of Ketoprofen in each example. The rotations of the product ethyl esters were measured at C = 1.0 in ethanol. The results are summarized in Table 5 below.

Table 5

| Example | Enzyme | % Conversion | α |
|---|---|---|---|
| 18 | Prozyme 6 | 3.7 | -0.166° |
| 19 | Protease 8 | 27.7 | +0.035° |
| 20 | Protease 27 | 26.9 | -0.203° |

This example shows that various hydrolase enzymes may be used in the practice of the separation process of the invention.

<u>Example 21</u>

Racemic Naproxen ethyl sulfate ester (sodium salt, 0.350 mol) were dissolved in one liter of 0.9 M sodium phosphate buffer, pH 7.0; made 10 v/v (2.5 M) in ethanol. This solution was recirculated through the lumen side of a SEPRACOR PAN fiber 0.85 $m^2$ bioreactor module. To extract the ethyl ester product formed by

enzymatic transesterification, 500 ml of toluene were circulated in a continuous loop through the shell side of the bioreactor module and through the shell side of a second identical SEPRACOR bioreactor module. The second module was provided in order to capture any Naproxen acid formed as a result of enzymatic hydrolysis of the ethyl sulfate ester substrate. Through the lumen of the second bioreactor module, 500 ml of 0.1 M sodium carbonate buffer, pH 10.0, was recirculated. The reservoir of the carbonate buffer recirculating through the second bioreactor was fitted with a pH stat, such that the reservoir could be continuously titrated with a 5 M sodium hydroxide solution to maintain the pH at 10.0. The entire apparatus was maintained at ambient temperature.

To initiate the resolution process, 20 g of the commerical protease Prozyme 6 were added to the phosphate buffer recirculating through the first bioreactor. The entire process was allowed to operate for 72 hours, after which time the total amount of acid titrated was 41.6 mmols. This indicated that 11.9% of the total starting material had been hydrolyzed to Naproxen acid. The phosphate buffer recirculating through the first bioreactor was then drained, and extracted four times with 250 ml of diethyl ether. The organic layers were combined with the toluene which had been recirculated through the two bioreactors. The entire organic phase was then dried over magnesium sulfate, and evaporated under reduced pressure to leave 43.8 g (172.4 mmols) of Naproxen ethyl ester, indicating that 49.3% of the total starting substrate had been transformed into ethyl ester. This material had a specific optical rotation of a -38.5°C at C = 1.0 in chloroform, indicating the presence of the (R) enantiomer in 79.8% enantiomeric excess.

The remaining aqueous reaction mixture was brought to pH 12.5 by the addition of sodium hydroxide, and allowed to stir for 4 hours. The pH was then lowered to approximately 3 by the addition of concentrated HCl, and the resulting mixture extracted four times with 250 ml of diethyl ether. The organic layers were combined, dried over magnesium sulfate, and evaporated under reduced pressure to leave Naproxen acid. This material had an optical rotation of +64.3°, indicating the presence of the (S) enantiomer in 96% enantiomeric excess. By subtracting the amount of Naproxen acid recovered in the previous step (41.6 mmols) and the amount of (R) ethyl ester formed (172.4 mmols), it was determined that 61% of the total starting substrate had been either transformed to (R)-Naproxen ethyl ester or hydrolyzed to Naproxen acid. Using 61% conversion and 96% e.e. of the remaining (S) enantiomer, an E value of 15.5 for this overall reaction was calculated.

Example 22

This example was conducted using the same apparatus and procedure as set forth in Example 21, with the following exceptions: a) 2 g of Prozyme 6 were used; b) total reaction time was 22 hours; c) 100 mmols of racemic Naproxen ethyl sulfate (sodium salt) were used; and d) sodium phosphate concentration in the buffer solution recirculating through the first bioreactor was 0.05 M. This experiment resulted in the production of 4.51 g of Naproxen acid (19.6 mmols), indicating that 19.6% of the total starting substrate had been hydrolyzed to Naproxen acid. 5.79 g (22.6 mmols) of Naproxen ethyl ester were recovered with a specific optical rotation of -35.4° at C = 1.0 in chloroform, indicating the presence of the (R) enantiomer in 73.3% enantiomeric excess. The combined amounts of these two products indicated that 42.5% of the total starting substrate had been either transformed to (R)-Naproxen ethyl ester of hydrolyzed to Naproxen acid.

The Naproxen acid recovered from base hydrolysis of the remaining aqueous buffer which had been recirculating the first bioreactor had a specific optical rotation of +39.6°, indicating the presence of (S)-Naproxen acid in 59.1% enantiomeric excess. Using a conversion of 59.1%, an E value of 17.6 was calculated for this experiment.

The invention may be embodied in other specific forms.

## Claims

1. A method of separating enantiomers comprising the steps of:

A. providing a mixture of (R) and (S) enantiomers of a water soluble reactant ester of the formula:

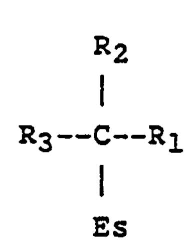

wherein Es is an ester group and $R_1$, $R_2$, and $R_3$ are different moities which together with the carbon atom define a chiral center;

B. reacting the mixture in a reaction solution comprising water, a hydrolase enzyme and a donor alcohol, at a concentration sufficient and for a time sufficient to transesterify preferentially at least a portion of one of said (R) and (S) enantiomers, to produce

a product ester of said one enantiomer, which product ester has reduced water solubility relative to said reactant ester, and

a residue of said reactant ester of the other enantiomer; and

C. separating from the reactant solution a fraction rich in one of said enantiomers.

**2.** The method of claim 1 wherein said reaction solution is a homogeneous aqueous solution of said hydrolase enzyme and said donor alcohol.

**3.** The method of claim 1 wherein said reaction solution comprises an aqueous solution of said donor alcohol and a separate hydrophobic phase for sequestering said product ester.

**4.** The method of claim 1 wherein the donor alcohol has the formula $R_4OH$ and the product ester is an $R_4$ ester, wherein $R_4$ is a hydrocarbon having 1 to 5 carbon atoms.

**5.** The method of claim 1 wherein the donor alcohol is a water soluble alcohol.

**6.** The method of claim 1 wherein the ratio of the water solubility of said reactant ester and said product ester is greater than about four.

**7.** The method of claim 1 wherein said product ester is substantially unreactive with respect to hydrolysis to its acid form by said hydrolase enzyme.

**8.** The method of claim 1 wherein the water soluble reactant ester has the formula:

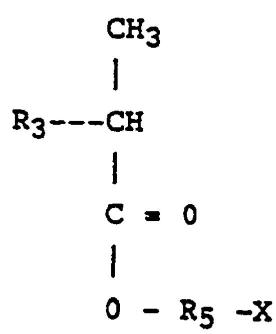

wherein $R_3$ is selected from the group consisting of halogen, aromatic ring structures, substituted aromatic ring structures, heterocyclic aromatic ring structures, substituted heterocyclic aromatic ring structures, aryloxy ring structures, and substituted aryloxy ring structures;

$R_5$ is a hydrocarbon having 1 to 8 carbon atoms; and

X is a hydrophilic moiety which imparts water solubility to said ester.

**9.** The method of claim 1 wherein $R_1$, $R_2$, and $R_3$, are each independently selected from the group consisting of hydrogen, halogen, lower alkyl, lower alkoxy, substituted lower alkyl, substituted lower alkoxy, hydroxy, thiol, nitrile, aromatic ring structures substituted aromatic ring structures, heterocyclic ring structures, aryloxy ring structures, and substituted aryloxy ring structures, and ES has the formula $COOR_5X$ wherein

$R_5$ is a hydrocarbon having 1 to 8 carbon atoms and

X is a hydrophilic moiety which imparts water solubility to said ester.

**10.** The method of claim 1 wherein said mixture of (R) and (S) reactant esters is a mixture of (R) and (S) 2-substituted propanoic acid ester.

**11.** The method of claim 1 wherein one of $R_1$ and $R_2$ is hydrogen and the other is methyl, and $R_3$ is selected

from the group consisting of:

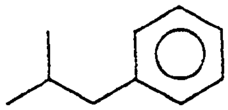 , 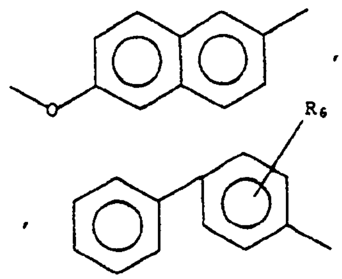 , 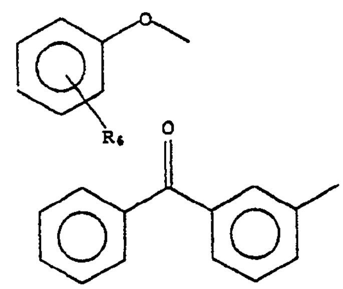

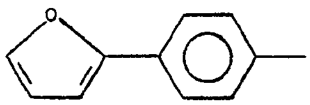 , and

wherein $R_6$ in H or halogen.

**12.** The method of claim 1 wherein said mixture of (R) and (S) reactant esters is a mixture of (R) and (S) 2-(p-isobutylphenyl) propanoic acid esters.

**13.** The method of claim 1 wherein said mixture of (R) and (S) reactant esters is a mixture of (R) and (S) 2-(6-methoxy-2-naphthyl) propanoic acid esters.

**14.** The method of claim 1 wherein said mixture of (R) and (S) reactant esters is a mixture of (R) and (S) 2–(aryloxy) propanoic acid esters or substituted 2-(aryloxy) propanoic acid esters.

**15.** The method of claim 8 or 9 wherein X is selected from the group consisting of quaternary amine, inorganic acid, and a salt thereof, and the carboxylic acid analog of said reactant ester is substantially water insoluble.

**16.** The method of claim 15 wherein X is a quaternary amine of the formula $-NZ_3$ wherein Z is selected from the group consisting of a hydrogen, alkyl, and aryl, and is charge balanced by a counterion.

**17.** The method of claim 16 wherein the counterion is selected from the group consisting of halide, carboxylate, inorganic polyatomic anions, methyl sulfate, tosylate, mesylate, and trifluoromethyl sulfonate.

**18.** The method of claim 8 or 9 wherein X is an inorganic acid selected from the group consisting of $-HSO_3$, $-HSO_4$, $-H_2PO_4$, and $-HPO_3$.

**19.** The method of claim 8 or 9 wherein X is a salt of an inorganic acid selected from the group consisting of $-SO_3^-$, $-SO_4^-$, $-HPO_4^-$, $-PO_4^=$, and $-PO_3^-$, and is charge balanced by a counterion.

**20.** The method of claim 1 wherein said hydrolase enzyme is selected from the group consisting of peptide hydrolases.

**21.** The method of claim 20 wherein said hydrolase enzyme is a serine protease.

**22.** The method of claim 21 wherein said protease enzyme is derived from Aspergillus oryzae.

**23.** The method of claim 1 wherein said hydrolase enzyme hydrolyzes preferentially a portion of said one enantiomer to produce an acid form of said one enantiomer, and the separating step comprises separating at least a portion of said acid form from said reactant ester.

**24.** The method of claim 1 wherein the separating step comprises partitioning a fraction rich in said product ester from said residue by extraction in hydrophobic media.

**25.** The method of claim 1 wherein the separating step comprises precipitating one of said reactant ester and said product ester.

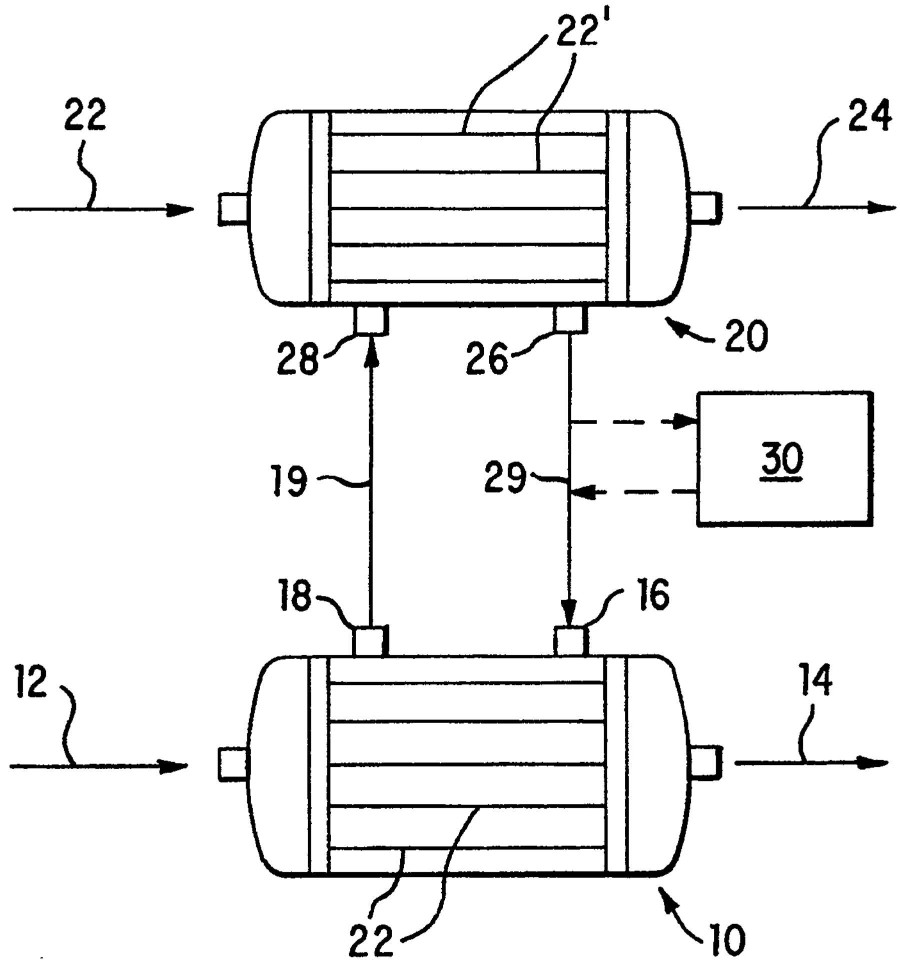
22'
22
24
20
28
26
19
29
30
18
16
12
14
22
10